# EUROPEAN PATENT APPLICATION

(11) **EP 4 497 423 A1**
(43) Date of publication of application: **29.01.2025**
(21) Application number: 24190076.0
(22) Date of filing: 22.07.2024
(51) Int. Cl.: A61F 13/15, A61F 13/53

(54) **APPARATUS AND METHOD FOR PRODUCING AN ABSORBENT SANITARY ARTICLE**

(30) Priority: 27.07.2023 IT 202300015846
(71) Applicant: GDM S.p.A., 40133 Bologna (IT)
(72) Inventor: ZAVALLONI, Alessandro, I-40133 Bologna (IT); TOSCANI, Federico, I-40133 Bologna (IT); ROSSI, Eros, I-40133 Bologna (IT)
(74) Representative: Porta & Consulenti Associati S.p.A.

(57) **Abstract**

The invention relates to an apparatus (100) for producing an absorbent sanitary article comprising a forming surface (124) at least partially permeable to air configured to move along a moving direction (D1) between a first forming position (F1), a second forming position (F2) and a third successive forming position (F3). The apparatus (100) comprises a first supply duct (131), configured to deliver loose absorbent material onto said forming surface (124) at said first forming position (F1), a second supply duct (132), configured to deliver loose absorbent material onto said forming surface (124) at said second forming position (F2), and a third supply duct (133), configured to deliver loose absorbent material onto said forming surface (124) at said third forming position (F3). The apparatus (100) further comprises a first suction chamber (141) opposite to said first supply duct (131) with respect to said forming surface (124) and arranged at said first forming position (F1), a second suction chamber (142) opposite to said second supply duct (132) with respect to said forming surface (124) and arranged at said second forming position (F2), and a third suction chamber (143) opposite to said third supply duct (133) with respect to said forming surface (124) at said third forming position (F3), said first suction chamber (141) being open on said forming surface (124) and configured to exert a suction through said forming surface (124), said second suction chamber (142) being open on said forming surface (124) and configured to exert a suction through said forming surface (124), said third suction chamber (143) being open on said forming surface (124) and configured to exert a suction through said forming surface (124).

The invention also relates to a method for producing an absorbent sanitary article.

## Description

The present invention relates to an apparatus and method for producing an absorbent sanitary article.

Absorbent sanitary articles are for example female sanitary napkins, baby diapers, napkins and pads for people suffering from incontinence and the like. In the following description, reference will be made to the non-limiting example of female sanitary napkins.

Structurally, a female sanitary napkin comprises a plurality of elements associated (glued and/or welded) together.

The female sanitary napkin is applied, by means of its first adhesive portions, to the inside of an undergarment worn by the user, by folding and flipping two opposite flaps on the outside of the garment and securing them to it with respective second adhesive portions.

Typically, the female sanitary napkin comprises a top sheet and a back sheet, overlapping and fastened to each other. An absorbent core is arranged between the top sheet and back sheet.

The top sheet is made of a material permeable to body fluids and is configured to face the user's body, in contact with it, when the sanitary towel is applied to the undergarment.

The absorbent core has the function of absorbing the body fluids that pass through the top sheet and of retaining them without leaks even for several hours. The absorbent core comprises an absorbent material typically in granular or powder form, called SAP (acronym for "Super Absorbent Powder"), capable of absorbing liquids up to 300 times its own weight and up to 60 times its own volume.

The back sheet is configured to be applied to the inside of the undergarment by the first adhesive portions and is made impermeable to body fluids to prevent leak of liquids absorbed by the absorbent core.

In markets that are more sensitive to environmental issues, lawmakers and consumers prefer biodegradable products.

Recently, biodegradable female sanitary napkins have been produced that can be disposed of in toilet bowls and dissolved in water within a period of days, weeks or months. In these products, in addition to making the top sheet and back sheet with biodegradable materials, the absorbent core is made from biodegradable SAP, such as carboxymethylcellulose (CMC).

Conventionally, in order to produce the absorbent core, loose fibres (e.g. cellulose) and SAP (e.g. granular sodium polyacrylate) are supplied onto an aspirated drum so as to compact the absorbent material.

The Applicant noted that biodegradable SAP powders generally have a smaller average grain size than conventional non-biodegradable SAPs, in particular even smaller than half, a third or a quarter of the grain size of conventional SAPs.

The Applicant found that, due to the fine particle size of biodegradable SAP, it is difficult to retain the fine particles within the cellulose fibre matrix and prevent the transfers of the semi-finished product and the suction vacuum from losing SAP during the production process.

The technical problem solved by the present invention is to provide an apparatus and method for producing an absorbent sanitary article that overcomes the aforementioned drawback.

The present invention therefore relates, in a first aspect thereof, to an apparatus for producing an absorbent sanitary article.

Preferably, a forming surface at least partially permeable to air is provided configured to move along a moving direction between a first forming position, a second forming position and a third successive forming position.

Preferably, a first supply duct is provided configured to deliver loose absorbent material on said forming surface at said first forming position.

Preferably, a second supply duct is provided configured to deliver loose absorbent material on said forming surface at said second forming position.

Preferably, a third supply duct is provided configured to deliver loose absorbent material on said forming surface at said third forming position.

Preferably, a first suction chamber is provided opposite to said first supply duct with respect to said forming surface.

Preferably, said first suction chamber is arranged at said first forming position.

Preferably, said first suction chamber is open on said forming surface and configured to exert a suction through said forming surface.

Preferably, a second suction chamber is provided opposite to said second supply duct with respect to said forming surface.

Preferably, the second suction chamber is arranged at said second forming position.

Preferably, said second suction chamber is open on said forming surface and configured to exert a suction through said forming surface.

Preferably, a third suction chamber is provided opposite to said third supply duct with respect to said forming surface.

Preferably said third suction chamber is arranged at said third forming position.

Preferably, said third suction chamber is open on said forming surface and configured to exert a suction through said forming surface.

In this description and in the following claims, loose absorbent material means loose fibres and/or powders of an absorbent material such as cellulose and biodegradable SAPs, e.g. carboxymethyl cellulose (CMC).

The term "loose" refers to a solid material consisting of elements without continuity between them, e.g. unconnected or weakly connected fibres, granules or particles.

"Air permeable" means a solid surface capable of being penetrated by atmospheric air, e.g. through holes arranged thereon, or a net structure.

In this description and in the following claims, terms referring to relative positions such as "upstream", "downstream", "successive" and the like, are to be understood as referring to the path of the loose absorbent material, unless otherwise specified.

The three supply ducts and the respective suction chambers allow three layers of absorbent material to be deposited on the forming surface, one on top of the other to form the absorbent core of the absorbent sanitary article, which is compacted by the suction exerted by the respective suction chambers, overcoming the drawback of the prior art.

Supplying absorbent material in the form of loose fibres only from the first supply duct creates a first layer of absorbent core of only fibres on the forming surface. The fibre size makes it possible to minimise or eliminate the amount of material passing through the forming surface and penetrating the first suction chamber.

Supplying absorbent material in the form of absorbent powder and, optionally, loose fibres from the second supply duct creates a second layer of absorbent core comprising absorbent powder overlapping the first layer of absorbent core. The first layer of absorbent core acts as a barrier when the second layer of absorbent core is formed to reduce or eliminate the amount of absorbent powder passing through the forming surface and penetrates the second suction chamber.

By supplying absorbent material in the form of loose fibres from the third supply duct, a third layer of absorbent core is created comprising compacted fibres overlapping the second layer of absorbent core. The first and third layers of absorbent core of compacted fibres cover the second layer of absorbent core and prevent absorbent power from escaping subsequently, such as during core transfers in the process of producing the absorbent sanitary article.

Furthermore, by producing the second layer of absorbent core with a high concentration of absorbent powder, in particular SAP, which, when absorbing liquids, turns into a gel, it is possible to achieve an effect known as *"gel blocking*" of body fluids, reducing the amount of liquid that can pass through the absorbent core and limiting the possibility of damage to the back sheet layer, which may be made of biodegradable material.

In a second aspect thereof, the present invention relates to a method for producing an absorbent sanitary article.

Preferably, a forming surface at least partially permeable to air is moved along a moving direction between a first forming position, a successive second forming position and a third forming position.

Preferably, loose absorbent material comprising loose fibres is delivered from a first supply duct onto said first forming surface at said first forming position.

Preferably, a suction is exerted from a first suction chamber, opposite to said first supply duct with respect to said forming surface and open on said forming surface, to produce a first layer of absorbent core of the absorbent sanitary article.

Preferably, the suction from the first suction chamber is exerted at the same time as the supply of loose absorbent material from the first supply duct.

Preferably, loose absorbent material comprising biodegradable absorbent powder is delivered from a second supply duct onto said first layer of absorbent core at said second forming position.

Preferably, a suction is exerted from a second suction chamber opposite to said second suction duct with respect to said forming surface, and open on said forming surface in order to produce a second layer of absorbent core.

Preferably, the suction from the second suction chamber is exerted at the same time as the supply of loose absorbent material from the second supply duct.

Preferably, loose absorbent material comprising loose fibres is delivered from a third supply duct onto said second layer of absorbent core at said third forming position.

Preferably, a suction is exerted from a third suction chamber opposite to said third supply duct with respect to said forming surface and open on said forming surface to produce a third layer of absorbent core.

Preferably, the suction from the third suction chamber is exerted at the same time as the supply of loose absorbent material from the third supply duct.

Preferably, the method of the second aspect of the present invention may be implemented by employing an apparatus according to the first aspect of the present invention.

The present invention may have, in both aspects discussed above, at least one of the preferred features described below. Such features may therefore be present individually or in combination, except where expressly stated otherwise, either in the method of the first aspect of the present invention or in the method of the second aspect of the present invention.

Preferably, a loose fibre supply member is provided configured to introduce loose fibre into said first, second and third supply ducts.

More preferably, the loose fibre supply member comprises a grinding mill configured to grind fibrous material to generate said loose fibres.

Preferably, the grinding mill is adjacent to respective side openings of said first, second and third supply ducts.

Preferably, an absorbent powder supply member is provided configured to introduce absorbent powder only into the second supply duct.

Preferably, said absorbent powder supply member comprises a hopper collecting absorbent powder from an industrial bag.

Preferably, said hopper is provided with a vibrating device.

Preferably, said hopper is provided with a stirrer, more preferably of the bridge-breaker type.

The hopper vibrating device and stirrer allow to improve the flow of absorbent powder, even with a very fine particle size, from the industrial bags (so-called "big bags"), preventing it from adhering to and sticking along the inner walls of the hopper.

Preferably, the absorbent powder supply member comprises a funnel located upstream of said second supply duct.

The funnel is preferably placed downstream of an auger dosing device configured to supply absorbent powder.

The auger dosing device is preferably connected to the hopper collecting absorbent material by a pneumatic conveyor.

Preferably, the auger dosing device is configured to deliver a predetermined dose of absorbent powder at each activation.

In a preferred embodiment of the invention, said forming surface comprises a plurality of recessed seats distributed along said moving direction.

Preferably, said recessed seats are equally spaced along said moving direction.

More preferably, said forming surface is permeable to air at said recessed seats.

A respective absorbent core of the absorbent sanitary article is formed in each recessed seat.

Preferably, a pick-up member is provided configured to pick up an absorbent core of the absorbent sanitary article from said forming surface at a release position downstream of said third forming position with respect to said moving direction.

The absorbent core then continues in the process of producing the absorbent sanitary article, to be interposed between the top sheet and the back sheet layer.

Preferably, a fourth suction chamber is provided opposite to said pick-up member with respect to said forming surface and extended along the moving direction between said third forming position and said release position.

Preferably, said fourth suction chamber is open on said forming surface and configured to exert a suction through said forming surface.

More preferably, a ventilation member is provided arranged downstream of said third forming position and configured to move an air flow against said forming surface along a direction opposite to said moving direction.

The action of the ventilation member improves the surface finish of the third layer of the absorbent core.

Preferably, said first, second and third supply ducts are defined in a box-shaped structure comprising two inner partition walls, the second supply duct being defined between the two partition walls.

More preferably, the second supply duct is placed at a median transverse plane of the box-shaped structure and the first and third supply ducts are substantially symmetrical to the median transverse plane.

Preferably, said forming surface is defined on a substantially cylindrical wall of a forming drum that is rotatable with respect to a rotation axis, wherein said moving direction is circumferential and said first, second and third forming positions are three consecutive angular positions.

The forming drum makes it possible to limit the overall size of the apparatus.

Preferably, said first, second and third suction chambers are contiguous and located in respective three circumferentially consecutive angular sectors defined radially inward from the substantially cylindrical wall of the forming drum.

Preferably, loose fibres are introduced into said first, second and third supply ducts by means of a loose fibre supply member.

Preferably, absorbent powder is introduced only into said second supply duct by an absorbent powder supply member.

Preferably, the absorbent core of the absorbent sanitary article is picked up from said forming surface at a release position located downstream of said third forming position with respect to said moving direction.

Preferably, the absorbent core is picked up from the forming surface by means of a pick-up member.

Preferably, suction is exerted from a fourth suction chamber, which is opposite to said pick-up member with respect to said forming surface and extending along the moving direction between said third forming position and said release position.

Preferably, downstream of said third forming position, an air flow is moved against said forming surface along a direction opposite to said moving direction.

Preferably, the air flow is moved by a ventilation member.

Further features and advantages of the present invention will become clearer from the following detailed description of a preferred embodiment thereof, with reference to the appended drawings and provided by way of indicative and non-limiting example, in which:
- Figure 1 is a front schematic view of an apparatus for producing an absorbent sanitary article according to the present invention;
- Figure 2 is a section schematic view of a part of the apparatus in Figure 1;
- Figure 3 is a front schematic view of a part of the apparatus according to the present invention arranged upstream of the one shown in Figure 1;
- Figure 4A is an exploded perspective schematic view of an absorbent sanitary article produced with the apparatus of the present invention;
- Figure 4B is a section schematic view of the absorbent sanitary article in Figure 4A.

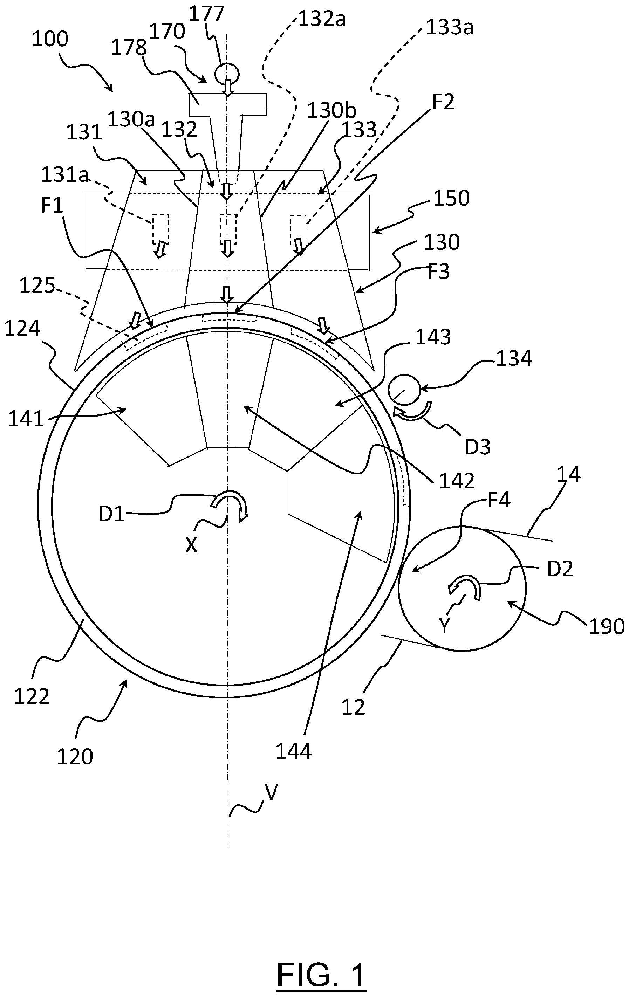

In Figures 1-3, reference number 100 is used to indicate an apparatus for producing an absorbent sanitary article according to the present invention.

Non-limiting examples of absorbent sanitary articles produced by the apparatus 100 are female sanitary napkins, baby diapers, napkins and pads for people suffering from incontinence and the like.

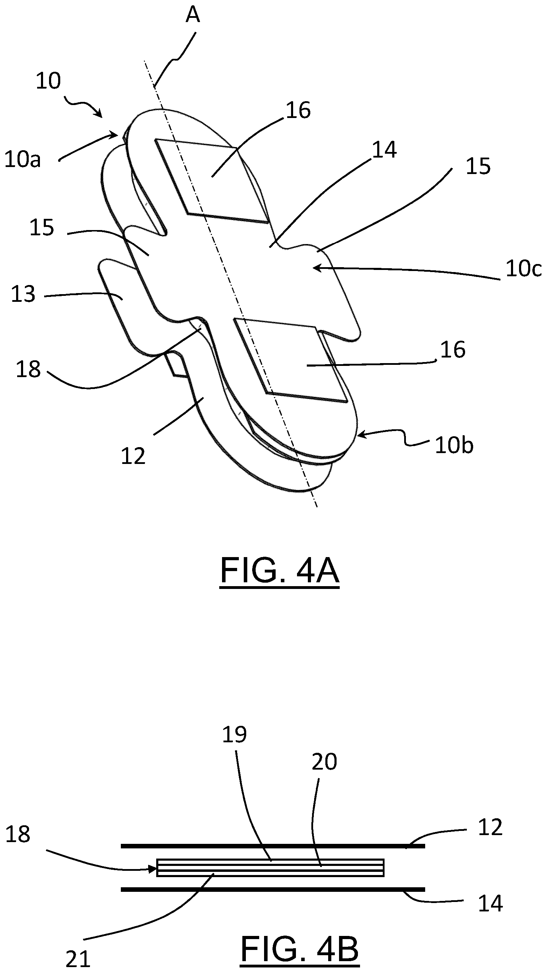

Without losing its generality, explicit reference will be made hereinafter to a specific example of an absorbent sanitary article, such as the one shown in Figures 4A and 4B and indicated by reference number 10. It is specifically a female sanitary napkin. What described, however, could find similar application to different types of absorbent sanitary articles, such as those listed above.

The article 10 has, along its longitudinal axis A, a front part 10a, a rear part 10b, and a central part 10c arranged between the front part 10a and the rear part 10b.

The article 10 comprises a plurality of elements that must be associated (glued and/or welded) with each other.

In particular, the article 10 comprises two primary elements extending longitudinally from the front 10a to the rear 10b of the article 10. More specifically, the aforementioned primary elements include a top sheet 12, and a back sheet 14. Both top sheet 12 and back sheet 14 are substantially rectangular in shape, with the two opposite lower sides rounded convexly and the two opposite upper sides rounded concavely. At the centre of each of the two opposite larger sides of the top sheet 12 and the back sheet 14 a fastening flap 13, 15 is provided which is cantilevered out from the larger side, transverse to the longitudinal axis A.

The top sheet 12 and back sheet 14 define, respectively, an inner face and an outer face of the article 10.

The article 10 is applied, by means of first adhesive portions 16 located on the back sheet 14, to the inside of an undergarment, by folding and flipping the two opposite flaps 13, 15 on the outside of the undergarment and attaching them to it with respective second adhesive portions (not shown).

The top sheet 12 overlaps the back sheet 14 and is configured to face the wearer's body when the article 10 is applied to the undergarment.

The top sheet 12 is made of a material permeable to body fluids. The back sheet 14 comprises a layer that is substantially impermeable to body fluids. In particular, the top sheet 12 and back sheet 14 are made of biodegradable materials, preferably cellulose fibres.

An absorbent core 18 is arranged between the top sheet 12 and the back sheet 14. This adsorbent core 18 has the function of absorbing the body fluids that pass through the top sheet 12 and of retaining them without leaks even for several hours. The absorbent core 18 is made of absorbent material comprising a fibre matrix, such as cellulose, and an absorbent powder (SAP) dispersed in the fibre matrix, such as carboxymethyl cellulose (CMC). The absorbent core 18 extends longitudinally from the front 10a to the rear 10b and is substantially oval in shape, narrowed at the centre 10c.

With reference to Figures 1-3, the apparatus 100 allows to produce the absorbent sanitary article 10.

The apparatus 100 comprises a forming drum 120 on which the absorbent core 18 is formed.

The forming drum 120 has an annular shape and comprises a substantially cylindrical wall 122 that is rotatable with respect to a rotation axis X.

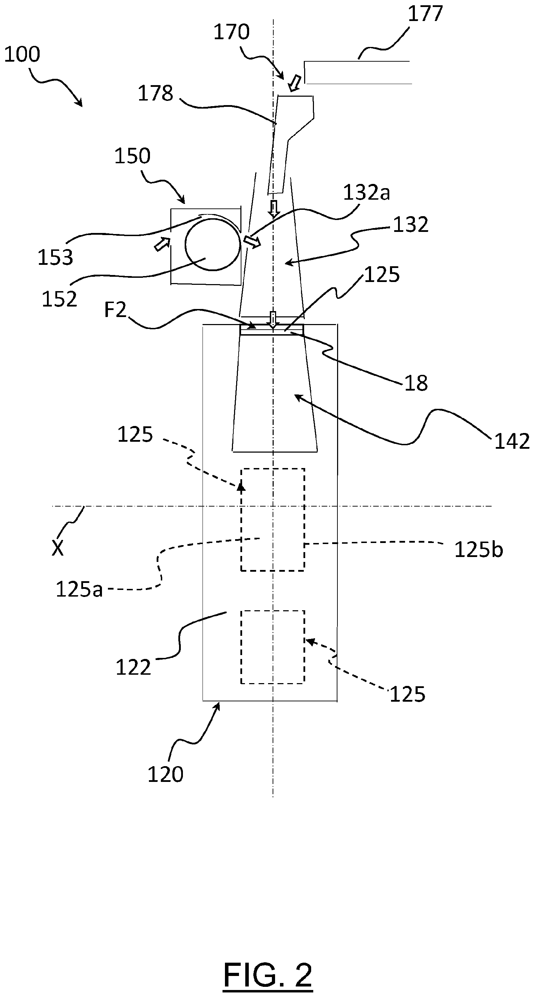

A forming surface 124 of the absorbent core 18 is defined on the substantially cylindrical wall 122. In particular, the forming surface 124 comprises a plurality of recessed seats 125, circumferentially distributed, preferably equally spaced-apart, around the rotation axis X. In each recessed seat 125, a respective absorbent core 18 of the article 10 is formed. Each recessed seat 125 comprises a substantially flat bottom wall 125a and a perimeter wall 125b substantially orthogonal to the bottom wall 125a. The perimeter wall 125b is substantially counter-shaped to the plan shape of the absorbent core 18. In Figure 2, the perimeter wall 125b is shown with a rectangular shape for the sake of simplicity.

The forming surface 124 is permeable to air at the recessed seats 125, in particular at the bottom walls 125a.

With the rotation of the forming drum 120 (in particular, clockwise in Figure 1), the forming surface 124 moves in a circumferential moving direction D1. By moving along the moving direction D1, each recessed seat 125 passes through, in succession, a first, a second and a third forming position F1, F2, F3, which correspond to three consecutive angular positions with respect to the rotation axis X. The three forming positions F1, F2, F3 are provided above the rotation axis X. The second forming position F2 is located at a vertical diametrical plane V of the forming drum 120 and the first forming position F1 and third forming position F3 are preferably mirrored to each other with respect to the vertical diametrical plane V.

Above the forming drum 120, a first supply duct 131 is provided configured to deliver loose absorbent material to a respective recessed seat 125 located at the first forming position F1.

Inside the forming drum 120 a first suction chamber 141 is provided. The first suction chamber 141 is located on the side opposite to the first supply duct 131 with respect to the recessed seat 125 placed at the first forming position F1.

The first suction chamber 141 is open on the recessed seat 125 at the first forming position F1 and exerts a suction through the forming surface 124, in particular through the recessed seat 125.

The first supply duct 131 delivers loose absorbent material comprising only loose fibres, particularly cellulose. The first suction chamber 141 simultaneously exerts a suction on the opposite side of the first supply duct 131 so as to attract and compact the loose absorbent material towards the bottom of the recessed seat 125. Thus, a first layer 19 of the absorbent core 18 of the article 10 is created, this first layer 19 comprising a compacted fibre matrix (Figure 4B).

Above the forming drum 120, a second supply duct 132 is provided that delivers loose absorbent material to the recessed seat 125 located at the second forming position F2. The second supply duct 132 is adjacent to the first supply duct 131 and successive to the first supply duct 131 with respect to the moving direction D 1.

Operationally, the second supply duct 132 delivers the loose absorbent material onto the recessed seat 125 which has already passed from the first forming position F1 where the first layer 19 of the absorbent core 18 was formed.

A second suction chamber 142 is located inside the forming drum 120 on the side opposite the second supply duct 132 with respect to the recessed seat 125 placed at the second forming position F2. The second suction chamber 142 is adjacent to the first suction chamber 141 and successive to the first suction chamber 141 with respect to the moving direction D1.

The second suction chamber 142 is open on the recessed seat 125 at the second forming position F2 and exerts a suction through the forming surface 124, in particular through the recessed seat 125.

The second supply duct 132 delivers loose absorbent material in the form of loose fibres, in particular cellulose fibres, and absorbent powder, in particular biodegradable SAP. The second suction chamber 142 simultaneously exerts a suction on the side opposite to the second supply duct 132 so as to attract and compact the loose absorbent material towards the bottom of the recessed seat 125. This creates a second layer 20 of the absorbent core 18 of the article 10, this second layer 20 comprising a compacted fibre matrix wherein the absorbent powder is dispersed (Figure 4B). The second layer 20 is formed directly on the already formed first layer 19.

Above the forming drum 120, a third supply duct 133 is provided that delivers loose absorbent material to the recessed seat 125 located at the third forming position F3. The third supply duct 133 is adjacent to the second supply duct 132 and successive to the second supply duct 132 with respect to the moving direction D1.

Operationally, the third supply duct 133 delivers the loose absorbent material to the recessed seat 125 which has already passed from the first forming position F1 and the second forming position F2 and where the first core layer 19 and the second core layer 20 have been formed.

A third suction chamber 143 is located inside the forming drum 120 on the side opposite to the third supply duct 133 with respect to the recessed seat 125 located at the third forming position F3. The third suction chamber 143 is adjacent to the second suction chamber 142 and successive to the second suction chamber 142 with respect to the moving direction D1.

The third suction chamber 143 is open on the recessed seat 125 located at the third forming position F3 and exerts a suction through the forming surface 124, in particular through the recessed seat 125.

The third supply duct 133 delivers loose absorbent material comprising only loose fibres, in particular cellulose. The third suction chamber 143 simultaneously exerts a suction on the side opposite to the third supply duct 133 so as to attract and compact the loose absorbent material towards the bottom of the recessed seat 125. Thus, a third layer 21 of the absorbent core 18 of the article 10 is produced, this third layer 21 comprising a matrix of compacted fibres (Figure 4B). The third layer 21 is formed directly on the already formed second layer 20.

As shown in Figure 4B, the absorbent core 18 thus formed is layer-shaped and comprises three overlapping layers 19, 20, 21: the first layer 19 of compacted fibres only, the second layer 20 of compacted fibres and absorbent powder material and the third layer 21 of compacted fibres only. The second layer 20 of compacted fibres and absorbent powder material is thus enclosed between the first layer 19 and the third layer 21 of compacted fibres only, avoiding dispersion of the absorbent powder material.

The first, second and third supply ducts 131, 132, 133 are adjacent to each other and are defined in a box-shaped structure 130 comprising two inner partition walls 130a, 130b. The box-shaped structure 130 is located above the forming drum 120.

The second supply duct 132 is defined between the two partition walls 130a, 130b and is located at a transverse median plane of the box-shaped structure 130, which substantially coincides with the vertical diametrical plane V. The first and third supply ducts 131, 133 are substantially symmetrical with respect to the vertical diametrical plane V.

As shown in Figures 1 and 2, the box-shaped structure 130 has a truncated pyramid shape and is open at the top and bottom.

The first, second and third supply chambers 141, 142, 143 are contiguous with each other and face respectively the first, second and third supply ducts 131, 132, 133.

In particular, the first, second and third suction chambers 141, 142, 143 are located in respective three circumferentially consecutive angular sectors defined radially inwardly with respect to the forming drum 120, in a radially inward position within the substantially cylindrical wall 122 of the forming drum 120.

The second suction chamber 142 is located at the vertical diametrical plane V. The first and third suction chambers 141, 143 are substantially symmetrical with respect to the vertical diametrical plane V.

The apparatus 10 comprises, at a release position F4 located downstream of the third forming position F3 with respect to the moving direction D1, a pick-up member 190 for picking up from each recessed seat 125 of the forming drum 120 the respective absorbent core 18 of the article 10. The pick-up member 190 is a roller 122 rotatable with respect to a rotation axis Y, in a circumferential pick-up direction D2 opposite to the moving direction D1 (specifically, counterclockwise in Figure 1). The pick-up member 190 is sucked in and located substantially tangent to the forming drum 120 at the release position F4.

In the process of producing the article 10, the absorbent core 18 is then interposed between the back sheet 14 and the top sheet 12. In the non-limiting case of Figure 1, the back sheet 14 is supplied on the pick-up member 190 itself, upstream of the release position F4 with respect to the pick-up direction D2, and the top sheet 12 is supplied on the pick-up member 190 itself, downstream of the release position F4 with respect to the pick-up direction D2. In this case, as shown in Figure 4B, the third layer 21 of the absorbent core 18 is in contact with the back sheet 14, while the first layer 21 of the absorbent core 18 is in contact with the top sheet 12.

The apparatus 100 comprises a fourth suction chamber 144 located inside the forming drum 120. The fourth suction chamber 144 is extended along the supply direction D1 between the third suction chamber 143 and the release position F4, on the side opposite to the pick-up member 190 with respect to the forming surface 124. The fourth suction chamber 144 is open on the forming surface 124 and configured to exert a suction through at least one recessed seat 125 located between the third forming position F3 and the release position F4. Operationally, this recessed seat 125 is passed from the first, second and third forming positions F1, F2, F3 and houses therein the three layers 19, 20, 21 of the absorbent core 18, overlapping each other.

The suction action exerted by the fourth suction chamber 144 is suitable to keep the layers 19, 20, 21 of the absorbent core 18 in the recessed seat 125 and preferably to compact them further.

The fourth suction chamber 144 ends at the release position F4. The suction action towards the forming surface 124 on the cores 18 formed in the recessed seats 125 thus ceases at the release position F4 and, at the same time, the suction action towards the pick-up member 190 begins.

The fourth suction chamber 144 is located in a respective further angular sector of the forming drum 120, circumferentially downstream of the three angular sectors wherein the first, second and third suction chambers 141, 142, 143 are located.

The apparatus 100 comprises a ventilation member 134 arranged downstream of the third forming position F3, radially external to the forming drum 120, to move an air flow against the recessed seat 125 located between the third forming position F3 and the release position F4. The air flow is directed in the direction D3 opposite to the moving direction D1, and is configured to flatten the third layer 21 of the absorbent core 18.

The apparatus 100 further comprises a loose fibre supply member 150 configured to introduce loose fibres, preferably cellulose, into the first, second and third supply ducts 131, 132, 133.

In particular, the loose fibre supply member 150 comprises a grinding mill 152 that grinds fibrous sheet material, preferably cellulose, in a duct 153 to generate loose fibre.

The grinding mill 152 is located next to the respective side openings 13 1a, 132a, 133a of the first, second and third supply ducts 131, 132, 133, through which the loose fibre is introduced.

Additionally, the apparatus 100 comprises an absorbent powder supply member 170 that only introduces absorbent powder of biodegradable SAPs into the second supply duct 132.

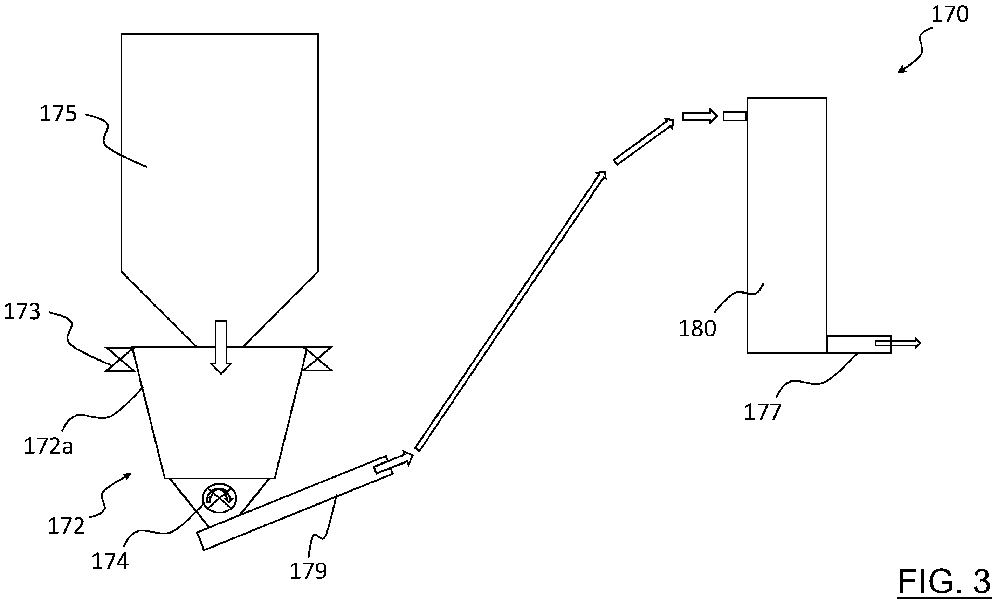

As shown in Figure 3, the absorbent powder supply member 170 comprises a hopper 172 collecting biodegradable SAP absorbent powder falling from an industrial bag 175.

The hopper 172 comprises a vibrating device 173 and a stirrer 174 with a so-called "bridge-breaker" movement system. The vibrating device 173 is preferably of the electromechanical type and is applied to an outer wall 172a of the hopper 172. The stirrer 174 is located in a lower portion of the hopper 172.

The hopper 172 is connected to an auger dosing device 177 (Figures 1-3) that supplies predetermined doses of biodegradable SAP absorbent powder to a funnel 178 (Figures 1 and 2) located at the inlet of the second supply duct 132.

In particular, the hopper 172 is connected to the auger dosing device 177 by means, in succession, of an auger extractor 179 and a pneumatic conveyor 180.

In an alternative embodiment of the invention, the forming surface could have a single recessed seat extending around the entire circumference of the forming drum. The continuous absorbent core deposited in such a recessed seat must be subsequently cut into portions of a predetermined length to produce absorbent sanitary articles according to the present invention.

Obviously, a person skilled in the art, in order to satisfy specific and contingent needs, can make numerous modifications and variations to the invention described above while remaining within the scope of protection defined by the following claims.

## Claims

1. Apparatus (100) for producing an absorbent sanitary article (10), comprising:
- a forming surface (124) at least partially permeable to air configured to advance along a moving direction (D1) between a first forming position (F1), a second forming position (F2) and a third forming position (F3) successive to each other;
- a first supply duct (131) configured to deliver loose absorbent material on said forming surface (124) at said first forming position (F1);
- a second supply duct (132) configured to deliver loose absorbent material on said forming surface (124) at said second forming position (F2);
- a third supply duct (133) configured to deliver loose absorbent material on said forming surface (124) at said third forming position (F3);
- a first suction chamber (141) opposite to said first supply duct (131) with respect to said forming surface (124) and arranged at said first forming position (F1), said first suction chamber (141) being open on said forming surface (124) and configured to exert a suction through said forming surface (124);
- a second suction chamber (142) opposite to said second supply duct (132) with respect to said forming surface (124) and arranged at said second forming position (F2), said second suction chamber (142) being open on said forming surface (124) and configured to exert a suction through said forming surface (124);
- a third suction chamber (143) opposite to said third supply duct (133) with respect to said forming surface (124) and arranged at said third forming position (F3), said third suction chamber (143) being open on said forming surface (F3) and configured to exert a suction through said forming surface (124).

2. Apparatus (100) according to claim 1, comprising a loose fibre supply member (150) configured to introduce loose fibres into said first, second and third supply ducts (131, 132, 133).

3. Apparatus (100) according to claim 1 or 2, comprising an absorbent powder supply member (170) configured to introduce absorbent powder only into said second supply duct (132).

4. Apparatus (100) according to claim 3, wherein said absorbent powder supply member (170) comprises a hopper (172) configured to collect absorbent powder from an industrial bag (175), said hopper (172) being provided with a vibrating device (173) and a stirrer (174).

5. Apparatus (100) according to any one of the previous claims, wherein said forming surface (124) comprises a plurality of recessed seats (125) distributed along said moving direction (D1), said forming surface (124) being permeable to air at said recessed seats (125).

6. Apparatus (100) according to any one of the previous claims, comprising a pick-up member (190) configured to pick an absorbent core (18) of the absorbent sanitary article (10) up from said forming surface (124) at a release position (F4) located downstream of said third forming position (F3) with respect to said moving direction (D1);
said apparatus (100) further comprising a fourth suction chamber (144) opposite to said pick-up member (190) with respect to said forming surface (124) and extending along the moving direction (D1) between said third forming position (F3) and said release position (F4), said fourth suction chamber (144) being open on said forming surface (124) and configured to exert a suction through said forming surface (124).

7. Apparatus (100) according to any one of the previous claims, comprising a ventilation member (134) arranged downstream of said third forming position (F3) and configured to move an air flow against said forming surface (124) along a direction opposite to said moving direction (D1).

8. Apparatus (100) according to any one of the previous claims, wherein said first, second and third supply ducts (131, 132, 133) are defined in a box-shaped structure (130) comprising two inner partition walls (130a, 130b), the second supply duct (132) being defined between the two partition walls (130a, 130b).

9. Apparatus (100) according to any one of the previous claims, wherein said forming surface (124) is defined on a substantially cylindrical wall (122) of a forming drum (120) rotatable about a rotation axis (X), wherein said moving direction (D1) is circumferential and said first, second and third forming positions (F1, F2, F3) are three consecutive angular positions.

10. Apparatus (100) according to claim 9, wherein said first, second and third suction chambers (141, 142, 143) are contiguous and located in respective three circumferentially consecutive angular sectors defined in a radially inner position with respect to said substantially cylindrical wall (122).

11. Method for producing an absorbent sanitary article (10), comprising:
- moving a forming surface (124), at least partially permeable to air, along a moving direction (D1) between a first forming position (F1), a second forming position (F2) and a third forming position (F3) successive to each other;
- delivering loose absorbent material comprising loose fibres from a first supply duct (131) onto said forming surface (124) at said first forming position (F1), and simultaneously exerting a suction from a first suction chamber (141) opposite to said first supply duct (131) with respect to said forming surface (124) and open on said forming surface (124), to obtain a first layer (19) of an absorbent core (18) of the absorbent sanitary article (10);
- delivering loose absorbent material comprising biodegradable absorbent powder from a second supply duct (132) onto said first layer (19) of the absorbent core (18) at said second forming position (F2) and simultaneously exerting a suction from a second suction chamber (142) opposite to said second supply duct (132) with respect to said forming surface (124) and open on said forming surface (124), to obtain a second layer (20) of the absorbent core (18);
- delivering loose absorbent material comprising loose fibres from a third supply duct (132) onto said second layer (20) of the absorbent core (18) at said third forming position (F3) and simultaneously exerting a suction from a third suction chamber (143) opposite to said third supply duct (133) with respect to said forming surface (124) and open on said forming surface (124), to obtain a third layer (21) of the absorbent core (18).

12. Method according to claim 11, comprising:
- introducing said loose fibres into said first, second and third supply ducts (131, 132, 133) by a loose fibre supply member (150);
- introducing said biodegradable absorbent powder only into said second supply duct (132) by an absorbent powder supply member (170).

13. Method according to claim 11 or 12, comprising:
- picking an absorbent core (18) of the absorbent sanitary article (10) up from said forming surface (124), by a pick-up member (190), at a release position (F4) located downstream of said third forming position (F3) with respect to said moving direction (D1).

14. Method according to claim 13, comprising:
- exerting a suction from a fourth suction chamber (144) opposite to said pick-up member (190) with respect to said forming surface (124) and extended along the moving direction (D1) between said third forming position (F3) and said release position (F4), said fourth suction chamber (144) being open on said forming surface (124).

15. Method according to any one of claims 11 to 14, comprising:
- moving, by a ventilation member (134) arranged downstream of said third forming position (F3), an air flow against said forming surface (124) along a direction opposite to said moving direction (D1).
